# EUROPEAN PATENT APPLICATION

(11) **EP 1 961 364 A1**
(43) Date of publication of application: **27.08.2008**
(21) Application number: 06823575.3
(22) Date of filing: 04.12.2006
(51) Int. Cl.: A61B 1/00

(54) **RECEIVING DEVICE**

(30) Priority: 05.12.2005 JP 2005350753
(71) Applicant: OLYMPUS MEDICAL SYSTEMS CORP., Shibuya-ku Tokyo 151-0072 (JP)
(72) Inventor: SHIGEMORI, Toshiaki c/o Olympus Medical Systems Corp., Tokyo 151-0072 (JP)
(74) Representative: von Hellfeld, Axel
(86) International application number: PCT/JP2006/324180
(87) International publication number: WO 2007/066620

(57) **Abstract**

Real-time observation of inside-subject images being examined by taking a body-insertable device and monitoring for malfunctions of a receiving device itself and the body-insertable device are enabled regardless of where the subject is located. By providing a wireless interface card (12) that enables radio communication with a mobile phone (8) having a display unit that can display images via a mobile phone communication network, a subject (1) being examined by taking a capsule endoscope (2) can perform radio communication between the receiving device (3) being carried by the subject (1) and the mobile phone (8) regardless of where the subject (1) is located to perform real-time observation of inside-subject images under examination and monitoring for malfunctions of the receiving device (3) and the capsule endoscope (2) through the display unit (9) of the mobile phone (8) regardless of where the subject (1) is located.

## Description

### TECHNICAL FIELD

The present invention relates to a receiving device that receives a radio signal transmitted from a body-insertable device such as a capsule endoscope moving inside the subject and can be carried in a subject.

### BACKGROUND ART

In recent years, a capsule endoscope equipped with an image pickup function and a radio communication function has entered a field of the endoscope. The capsule endoscope is adopted to move inside organs such as the esophagus, stomach, and small intestine (inside body spaces) during an observation period after being swallowed through a mouth of a subject, which is a subject, for observation (examination) until naturally defecated from the subject (human body) with peristaltic movement to successively pick up images at a predetermined route for image pickup using the image pickup function.

Image data picked up by the capsule endoscope inside body spaces during the observation period while moving inside such organs is transmitted to outside a subject by the radio communication function such as sequential radio communication to be stored in memory provided inside an external receiver. With a receiving device with the radio communication function and memory function being carried by a subject, the subject can move freely without mobility impairment even in an observation period till defecation after a capsule endoscope is swallowed.

To receive image data, a receiver generally has a plurality of antennas, distributed outside the subject, to receive an image signal transmitted from a capsule endoscope and receives image signal while switching to an antenna of high receiving intensity. In Patent Document 1, for example, a receiver is described that switches reception of a plurality of antennas arranged outside a subject and based on field intensity received by each antenna, detects a location of a capsule endoscope, which is a source of image signal, inside the subject.

In such a capsule endoscope system, an image inspection is generally carried out after a series of operations to pick up images by the capsule endoscope is completed by transmitting image data stored in a memory of the receiver to a workstation or the like. However, there is a strong desire among physicians and the like to carry out an inspection of picked-up images in real time, and a system with a simplified image display device, which displays images in real time based on a radio signal transmitted from a capsule endoscope, is has been proposed.

A conventional simplified image display device is, in its simplest configuration, adopted to allow electrical connection to a receiver and is equipped with a small display unit and a predetermined signal processing unit. With such a configuration, it becomes possible to input a signal subjected with reception processing at a receiver into the simplified image display device to display an image picked up by a capsule endoscope on the small display unit after performing predetermined processing based on an input signal. With such a configuration, picked-up images can be observed in real time.

Patent Document 1: Japanese Patent Application Laid-Open No. 2003-19111

### DISCLOSURE OF INVENTION

### PROBLEM TO BE SOLVED BY THE INVENTION

However, such a simplified image display device assumes, for example, a real-time observation in a laboratory where a physician can be close by a subject and if the subject being examined by taking a capsule endoscope acts freely at another place in a hospital or leaves a hospital building after leaving the laboratory, the physician and the like cannot observe images picked up inside body spaces in real time. Though real-time observation with the simplified image display device can be continued by locking the subject in the laboratory, since an examination using a capsule endoscope usually lasts more than eight hours, merits of an examination system using a capsule endoscope will be reduced by half if location or behavior of the subject is constrained. Also, if the subject always carries a portable simplified image display device together with a receiver, images picked up inside body spaces can be observed in real time at a desired time as needed, but it is troublesome for the subject to always carry a dedicated simplified image display device in addition to a required receiver. Moreover, physicians and the like strongly desire to monitor for any malfunction of the receiver being carried by the subject who went out and the capsule endoscope swallowed by the subject, but currently there is no other means than to rely on reports from the subject.

The present invention has been developed in consideration of the above-described circumstances and an object thereof is to provide a receiving device that allows real-time observation of images inside a subject under examination with a body-insertable device and monitoring for malfunctions of the receiving device itself and the body-insertable device, regardless of where the subject is located.

### MEANS FOR SOLVING PROBLEM

To solve the problems and achieve the object, a receiving device as set forth in claim 1 can receive a radio signal transmitted from a body-insertable device moving inside the subject and allow the subject to carry. The receiving device includes a radio communication interface that enables radio communication via a radio communication network with a communication terminal device having a display unit capable of displaying an image.

In the receiving device as set forth in claim 2 according to the above invention, the radio communication interface is a mobile phone interface.

In the receiving device as set forth in claim 3 according to the above invention, the radio communication interface is a wireless LAN interface.

The receiving device as set forth in claim 4 according to the above invention includes a mounting unit to which the radio communication interface adapted to a type of target communication terminal device can freely be mounted.

The receiving device as set forth in claim 5 according to the above invention includes a transmission/output unit that transmits/outputs inside-subject image data picked up by the body-insertable device, to the communication terminal device via the radio communication interface with an access from the communication terminal device.

The receiving device as set forth in claim 6 according to the above invention includes an access unit that accesses the communication terminal device of a preset specific address; and a transmission/output unit that transmits/outputs inside-subject image data picked up by the body-insertable device to the communication terminal device via the radio communication interface during an access by the access unit.

In the receiving device as set forth in claim 7 according to the above invention, the access unit accesses the communication terminal device of the specific address at specific timing.

In the receiving device as set forth in claim 8 according to the above invention, the specific timing is periodic timing.

The receiving device as set forth in claim 9 according to the above invention includes an organ determination unit that determines any change of an imaged organ by performing image processing of inside-subject image data picked up by the body-insertable device, wherein the specific timing is a timing when the organ imaged by the body-insertable device changes according to a determination of the organ determination unit.

The receiving device as set forth in claim 10 according to the above invention includes a lesion area determination unit that determines whether there is any lesion area by performing image processing of inside-subject image data picked up by the body-insertable device, wherein the specific timing is a timing when a lesion area is detected according to a determination of the lesion area determination unit.

The receiving device as set forth in claim 11 according to the above invention includes a manual operation button for instructing transmission/output of inside-subject image data, wherein the specific timing is a timing when the manual operation button is operated.

The receiving device as set forth in claim 12 according to the above invention includes an access unit that accesses the communication terminal device of a preset specific address; and a warning output unit that transmits/outputs warning information to the communication terminal device via the radio communication interface during access by the access unit.

The receiving device as set forth in claim 13 according to the above invention includes a malfunction detection unit that detects whether there is any malfunction of the receiving device, wherein the access unit accesses the communication terminal device of the specific address when a malfunction is detected by the malfunction detection unit, and the warning output unit transmits/outputs warning information, indicating that the receiving device malfunctions, to the communication terminal device.

The receiving device as set forth in claim 14 according to the above invention includes a malfunction detection unit that detects whether there is any malfunction of the body-insertable device, wherein the access unit accesses the communication terminal device of the specific address when a malfunction is detected by the malfunction detection unit, and the warning output unit transmits/outputs warning information, indicating that the body-insertable device malfunctions, to the communication terminal device.

A receiving device as set forth in claim 15 can receive a radio signal transmitted from a body-insertable device moving inside the subject and can allow the subject to carry. The receiving device includes a connection port for connecting a mobile phone via a cable; and an output unit that transmits/outputs inside-subject image data picked up by the body-insertable device to the mobile phone via the connection port when the mobile phone is connected to the connection port.

### EFFECT OF THE INVENTION

Since a receiving device according to the present invention comprises a radio communication interface that enables radio communication via a radio communication network with a communication terminal device having a display unit that can display images, radio communication between a receiving device carried by a subject and the communication terminal device can be performed regardless of the location of the subject being examined with a body-insertable device swallowed and thus advantageous effects of, being able to perform real-time observation of inside-subject images under examination and monitoring for malfunctions of the receiving device itself and the body-insertable device via the display unit of the communication terminal device regardless of where the subject is located, can be obtained.

Also, since a receiving device according to the present invention includes a connection port for connecting a mobile phone via a cable and an output unit for transmitting/outputting inside-subject image data picked up by a body-insertable device to the mobile phone via the connection port when the mobile phone is connected to the connection port, the subject can perform communication, by using the mobile phone owned by the subject, between the receiving device being carried and the mobile phone of the subject regardless of the location of the subject being examined by with the body-insertable device swallowed and thus advantageous effects, of being able to perform real-time observation of inside-subject images under examination via the display unit of the mobile phone of the subject regardless of the location, can be obtained.

### BRIEF DESCRIPTION OF DRAWINGS

FIG. 1 is a schematic diagram of an overall configuration of a wireless type body-insertable device system, which is a suitable embodiment of a receiving device according to the present invention;
FIG. 2 is a schematic diagram showing the configuration of the receiving device that can communicate with a mobile phone by radio;
FIG. 3 is a schematic block diagram showing the configuration of the receiving device;
FIG. 4 is an outline flowchart showing a processing control example when the receiving device is accessed by the mobile phone;
FIG. 5 is an outline flowchart showing a processing control example when accessing at preset periodic timing accompanying periodic output processing;
FIG. 6 is an outline flowchart showing a processing control example when accessing at timing when an imaged organ changes accompanying output processing for each organ;
FIG. 7 is an outline flowchart showing a processing control example when accessing at timing when a lesion area is detected accompanying output processing for each lesion area;
FIG. 8 is an outline flowchart showing a processing control example when accessing at timing when a manual operation button of the receiving device is pressed accompanying output processing for instruction operations;
FIG. 9 is an outline flowchart showing a processing control example when accessing at timing when a malfunction is detected in the receiving device accompanying output processing when a receiver malfunctions;
FIG. 10 is an outline flowchart showing a processing control example when accessing at timing when a malfunction is detected in a capsule endoscope accompanying output processing when a capsule malfunctions;
FIG. 11 is a schematic diagram showing the configuration of a receiving device that can communicate by radio of a Modification 2; and
FIG. 12 is an outline flowchart showing an output processing example when connected via a cable.

### EXPLANATIONS OF LETTERS OR NUMERALS

- 1: Subject
- 2: Capsule endoscope
- 3: Receiving device
- 7: Mobile phone communication network
- 8: Mobile phone
- 9: Display unit
- 11: Slot
- 12: Wireless interface card
- 16: Manual operation button
- 31: Transmission/output unit
- 32: Warning output unit
- 33: Access unit
- 34: Organ determination unit
- 35: Lesion location determination unit
- 36: Receiver malfunction detection unit
- 37: Capsule malfunction detection unit
- 41: Wireless LAN
- 42: Wireless LAN interface
- 43: Display unit
- 44: Notebook computer
- 45: Display unit
- 46: PDA
- 51: Mobile phone
- 52: USB cable
- 53: USB port

### BEST MODE(S) FOR CARRYING OUT THE INVENTION

An embodiment of a receiving device according to the present invention will be described in detail below based on drawings. However, the present invention is not limited to the embodiment and can be carried out in various modifications without departing from the spirit of the present invention.

### (Embodiment)

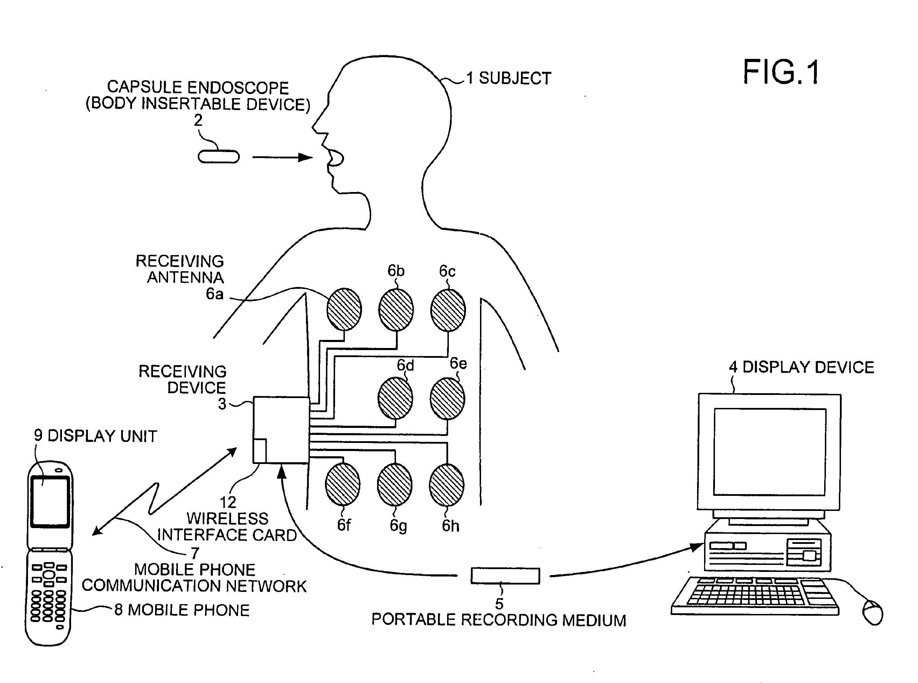

FIG. 1 is a schematic diagram of an overall configuration of a wireless type body-insertable device system, which is a suitable embodiment of a receiving device according to the present invention. As shown in FIG. 1, the body-insertable device system according to the present embodiment includes a capsule endoscope 2 that moves along a passing route after being introduced into a subject 1, a receiving device 3 that receives a radio signal including inside-subject information transmitted from the capsule endoscope 2, a display device 4 that displays contents of the inside-subject information included in the radio signal received by the receiving device 3, a portable recording medium 5 for transferring information between the receiving device 3 and the display device 4, and a mobile phone 8 that performs radio communication with the receiving device 3 via a mobile phone communication network 7 if necessary.

The display device 4 is used to display inside-subject images and the like picked up by the capsule endoscope 2 and received by the receiving device 3 and the like, and has a configuration like a workstation that displays images based on data obtained from the portable recording medium 5. More specifically, the display device 4 may be adapted to directly display images and the like on a CRT display and a LCD or the like, or adapted to output images to another medium like a printer.

The portable recording medium 5 is detachably attached to the receiving device 3 and the display device 4 and is adapted to enable output and recording of information when inserted in both devices. More specifically, while the capsule endoscope 2 is moving in body spaces of the subject 1, the portable recording medium 5 is adapted to be inserted in the receiving device 3 to store inside-subject images. Then, after the capsule endoscope 2 is discharged from the subject 1, the portable recording medium 5 is adapted to be removed from the receiving device 3 and insertable into the display device 4 so that recorded data is read by the display device 4. In contrast to a case in which the receiving device 3 and the display device 4 are connected by a cable, by using the portable recording medium 5 such as Compact Flash (registered trademark) memory to transfer data between the receiving device 3 and the display device 4, the subject 1 can move freely even while the capsule endoscope 2 is moving inside the subject 1.

Receiving antennas 6a to 6h are formed by using, for example, a loop antenna. The loop antenna is used while fixed at a predetermined location on a body surface of the subject 1 and the receiving antennas 6a to 6h preferably include a fixing means for fixing the loop antenna to the body surface of the subject 1.

The capsule endoscope 2 functions as an example of a body-insertable device and is used, driven by a battery, to pick up inside-subject images and to transmit a radio signal including picked-up image data to the receiving device 3. That is, the capsule endoscope 2 obtains inside-subject images within the subject, which are images of inside the subject 1, and intermittently transmits a radio signal including obtained image data to outside the subject until the capsule endoscope 2 is defecated after being introduced via an oval cavity of the subject 1.

Next, the receiving device 3 will be described. The receiving device 3 is used to receive a radio signal transmitted from the capsule endoscope 2 to reconstruct data related to inside-subject images contained in the radio signal. The receiving device 3 is also used, when necessary, to perform radio communication with the mobile phone 8 owned by a health care professional such as a doctor in charge, a nurse or a subject himself (herself) and having a display unit 9 that can display images such as an LCD.

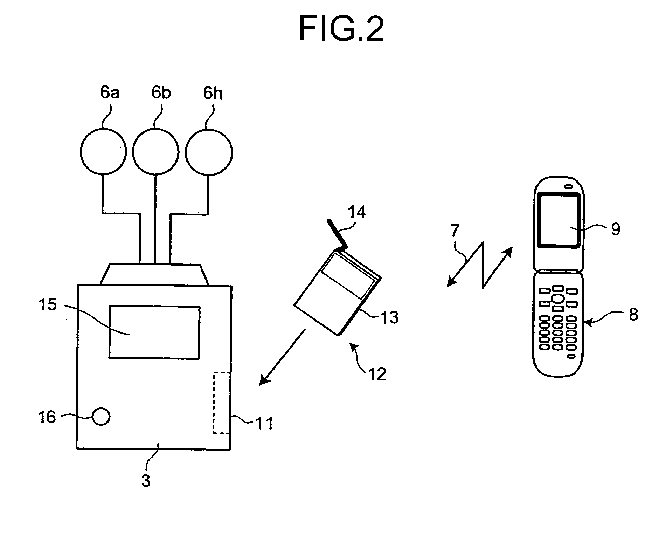

FIG. 2 is a schematic diagram showing the configuration of the receiving device 3 that can perform radio communication with the mobile phone 8. As shown in FIG. 2, the receiving device 3 of the present embodiment has a slot 11 compatible with a Compact Flash (registered trademark) memory card (CF card) to interface radio communication with the mobile phone 8 and a wireless interface card 12 compatible with the CF card with a mail address assigned as a specific address, which enables radio communication with the mobile phone 8 via the mobile phone communication network 7, is inserted in the slot 11. The wireless interface card 12 is included of a card-formed mobile phone interface circuit 13 and a radio antenna 14. More specifically, the wireless interface card 12 is intended for FORM (Freedom Of Mobile multimedia Access) and the mobile phone communication network 7 is, for example, a FORM communication network of 384 kbps (downstream) and 64 kbps (upstream). As shown in the FIG. 2, the receiving device 3 includes a small display unit 15 used for initializing ID information and the like for the receiving device 3 and a manual operation button 16 for instructing wireless transmission to the mobile phone 8 when desired by the subject himself (herself).

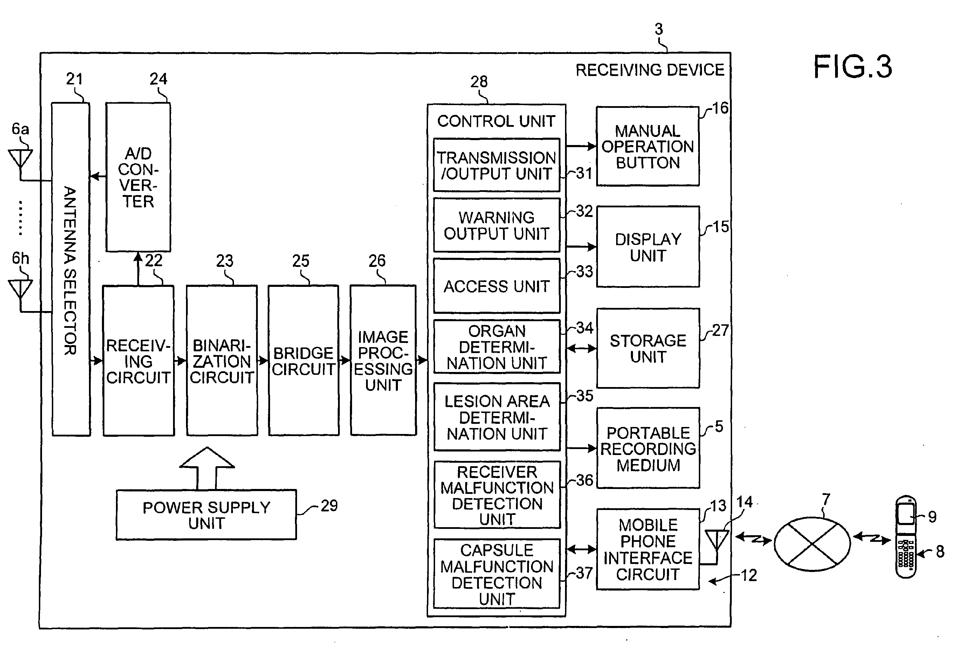

FIG. 3 is a schematic block diagram showing the configuration of the receiving device 3. As shown in the FIG. 3, the receiving device 3 includes an antenna selecting unit 21 that selects an antenna most suitable for receiving a radio signal among a plurality of existing receiving antennas 6a to 6h, a receiving circuit 22 that performs processing such as demodulation of the radio signal received via the receiving antenna 6 selected by the antenna selecting unit 21, a binarization circuit 23 that converts an original signal, (a signal before being modulated by the transmission unit 9) extracted by.the receiving circuit 22, into a digital signal that takes a binary value, and an A/D conversion unit 24 that converts a receiving intensity signal output from the receiving circuit 22 into a predetermined digital signal. The receiving device 3 also includes an image processing unit 26 that reconstructs inside-subject image data based on an original signal binarized by the binarization circuit 23 and passed through a bridge circuit 25, the portable recording medium 5 as memory to store image data reconstructed by the image processing unit 26, the wireless interface card 12, the display unit 15, and the manual operation button 16. Further, the receiving device 3 of the present embodiment includes a storage unit 27, a control unit 28 controlling input/output with respect to the portable recording medium 5, wireless interface card 12, display unit 15, manual operation button 16, and storage unit 27, and a battery-formed power supply unit 29 that supplies driving power to each component of the receiving device 3.

The antenna selecting unit 21 is used to select an antenna most suitable for reception among the plurality of receiving antennas 6a to 6h and to output a radio signal received via the selected receiving antenna to the receiving circuit 22. More specifically, for example, the antenna selecting unit 21 receives a radio signal by switching the receiving antennas 6a to 6h successively in advance and outputs the received radio signal to the receiving circuit 22. The receiving circuit 22 has a function to output an analog signal of RSSI (Received Signal Strength Indicator) to the A/D conversion unit 24 and the A/D conversion unit 24 converts an analog signal input from the receiving circuit 22 into a digital signal to output the digital signal to the antenna selecting unit 21. Then, the antenna selecting unit 21 selects a receiving antenna via which an RSSI digital signal input from the A/D conversion unit 24 becomes the strongest and outputs receipt of a radio signal received via the selected receiving antenna to the receiving circuit 22. Incidentally, the receiving antenna most suitable for reception changes over time as the capsule endoscope 2 moves inside the subject 1. Therefore, an antenna selection operation by the antenna selecting unit 21 is preferably performed multiple times.

The receiving circuit 22 is used to extract an original signal by performing processing such as demodulation of a received radio signal. In the present embodiment, the receiving circuit 22 is to be used to extract an original signal of an analog signal and to output it. The extracted original signal is converted into a digital signal by the binarization circuit 23 and the digital signal is subjected to serial/parallel conversion processing by the bridge circuit 25 before being output to the image processing unit 26.

The image processing unit 26 is used to reconstruct image data related to inside-subject images based on the original signal output from the binarization circuit 23 and includes a compression circuit for compressing, for example, in JPEG image data, and further includes a memory system such as SDRAM (Synchronous Dynamic Random Access Memory) for temporarily holding data.

The storage unit 27 is used to store initialization information and the like necessary for the receiving device 3 and, the storage unit 27 is set and registered with a mail addresses (specific addresses) of the mobile phones 8 owned by health care professionals in charge and the patient himself (herself) as well as a patient ID and the like.

The control unit 28 for controlling input/output into/from the portable recording medium 5 and the like includes a transmission/output unit 31, a warning output unit 32, an access unit 33, an organ determination unit 34, a lesion area determination unit 35, a receiver malfunction detection unit 36, and a capsule malfunction detection unit 37. The transmission/output unit 31 is used to transmit/output inside-subject image data picked up by the capsule endoscope 2 to the predetermined mobile phone 8 via the wireless interface card 12 and the mobile phone communication network 7 when accessed from the mobile phone 8 or the predetermined mobile phone 8 is accessed and the transmission/output unit 31 includes, for example, a function to decompress, modulate, and output compressed image data.

The organ determination unit 34 is used to determine in real time any change of an imaged organ based on inside-subject image data picked up by the capsule endoscope 2 and for which image processing has been performed. Relating to organs of the stomach, small intestine, and large intestine, for example, the organ determination unit 34 has a function to determine that the first area is the stomach, then determines that the capsule endoscope 2 has reached the small intestine area if a villous area, which is characteristic of a small intestine area, is detected based on inside-subject image data, and further, determines the capsule endoscope 2 has reached the large intestine area if a stool area, which is characteristic of a large intestine area, is detected based on inside-subject image data.

The lesion area determination unit 35 is used to determine in real time whether there is any lesion area (bleeding, discoloration, abnormal shape and so on) based on inside-subject image data picked up by the capsule endoscope 2 and for which image processing has been performed. A bleeding area, for example, can be determined based on an occurrence of a more reddish image area than a mucosa inside an organ.

The receiver malfunction detection unit 36 is used to detect whether there is any malfunction or failure of the receiving device 3 itself. Malfunctions or failures in this case include, for example, whether batteries serving as the power supply unit 29 have run out, whether the portable recording medium 5 is securely inserted, whether a medium is normal, whether there is sufficient free space, and whether an examination ID is registered. For example, whether batteries have run out can be determined based on measurement of a battery voltage value. Whether the medium is normal can be determined based on whether data can be written into the portable recording medium 5 and whether the examination ID or the like is registered can be determined based on whether the predetermined data such as examination date, name of a subject, capsule ID, and lot number are registered in the portable recording medium 5.

The capsule malfunction detection unit 37 is used to detect whether the capsule endoscope 2 is malfunctioning. Malfunctions or failures in this case include, for example, whether batteries have run out, whether an LED is lighting normally, whether a CCD picks up images correctly, and image data is output normally to the receiving device 3 by radio. For example, whether LED or CCD is working normally can be determined based on content of image data received by the receiving device 3.

The warning output unit 32 is used to output warning information indicating that the receiving device 3 or the capsule endoscope 2 is malfunctioning to the specific mobile phone 8 set and registered in advance via the wireless interface card 12 and mobile phone communication network 7 when a malfunction is detected by the receiver malfunction detection unit 36 or capsule malfunction detection unit 37, and the warning information is set and registered in the storage unit 27 in advance.

The access unit 33 is used to access the mobile phone 8 of a mail address (specific address) set and registered in the storage unit 27 in advance from the receiving device 3 and performs access processing at specific timing. Processing examples, in which timing arises for the access unit 33 in the present embodiment to perform access processing, include periodic output processing, output processing for each organ, output processing for each lesion area, output processing for instruction operations, output processing when a receiver malfunctions, and output processing when a capsule malfunctions. Each processing will be described later.

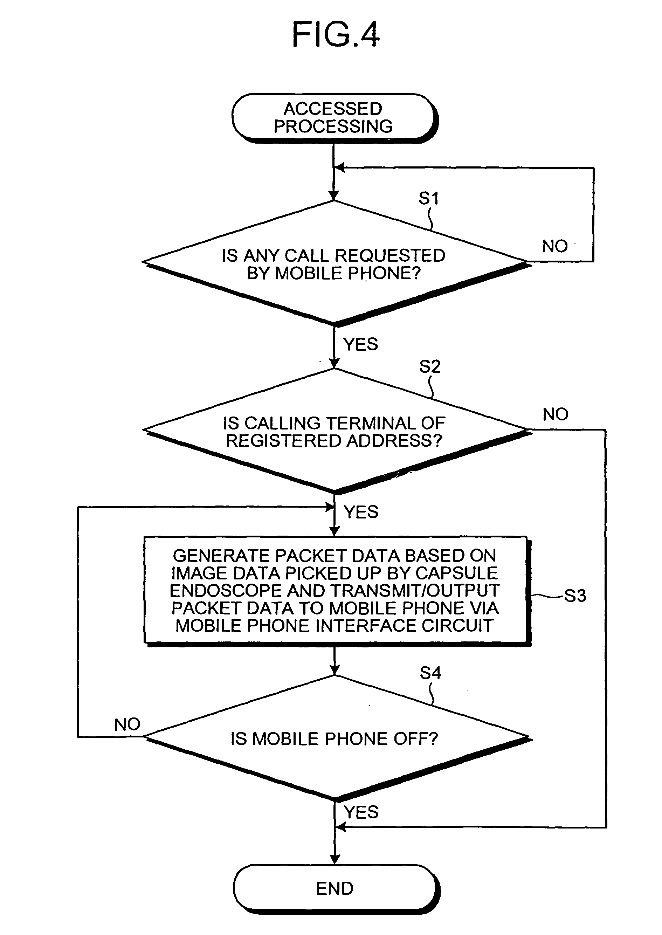

Next, examples of radio communication processing control with the mobile phone 8 performed by the control unit 28 will be described. First, a processing control example when the receiving device 3 is accessed by the mobile phone 8 will be described. FIG. 4 is an outline flowchart showing a processing control example when the receiving device 3 is accessed by the mobile phone 8. If a call is requested by the mobile phone 8 by specifying an address of the receiving device (step S1, Yes), it is determined whether the calling mobile phone 8 that requested the call is a mobile phone of an address already set and registered in the storage unit 27 (step S2). If the calling mobile phone 8 is not registered (step S2, No), processing is terminated, and if the calling mobile phone 8 is registered (step S2, Yes), the transmission/output unit 31 generates packet data based on inside-subject image data picked up by the capsule endoscope 2 to transmit/output the packet data to the calling mobile phone 8 via the mobile phone interface circuit 13, antenna 14, and mobile phone communication network 7 (step S3). This makes it possible to display inside-subject images picked up by the capsule endoscope 2 on the display unit of the mobile phone 8 in real time. Such transmission/output of packet data is repeated until a calling state is turned off by the mobile phone 8 (step S4, Yes).

According to this example, a health care professional in charge can perform real-time observation of inside-subject images under examination through the display unit 9 of the mobile phone 8 by accessing the receiving device 3 carried by a subject by performing a remote operation from inside a hospital at a desired timing using the mobile phone 8 owned by the health care professional, regardless of the location of the subject being examined by swallowing the capsule endoscope 2. Particularly, it becomes possible to perform real-time observation of inside-subject images of a subject under examination of the small intestine who has permission to leave the hospital building. Also, the subject himself (herself) can always perform real-time observation of inside-subject images under examination through the display unit 9 of the mobile phone 8 by accessing the receiving device 3 being carried by the subject from the mobile phone 8 owned by the subject. Nowadays, the mobile phone 8 has become an indispensable device for everyday life and, by simply carrying the mobile phone 8 and receiving device 3, real-time observation by the subject himself (herself) becomes possible.

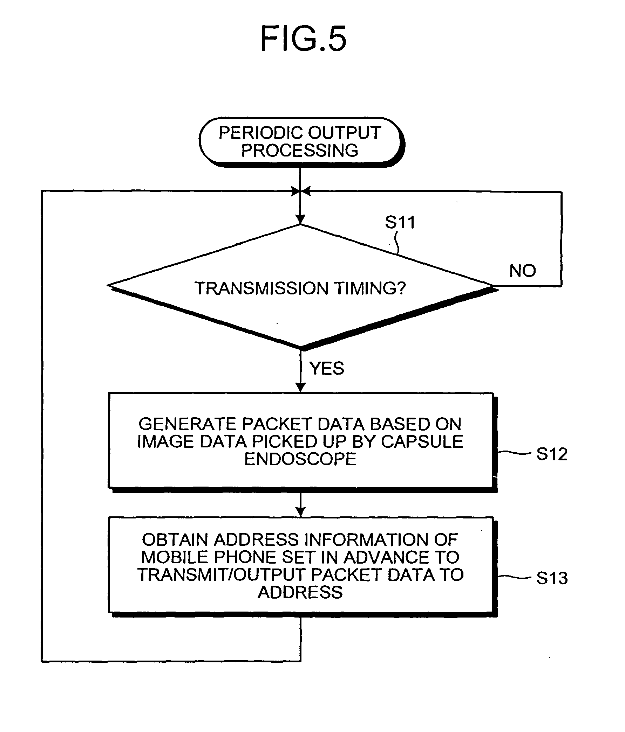

A processing control example when the specific mobile phone 8 is accessed at specific timing by the receiving device 3 will be described. FIG. 5 is an outline flowchart showing a processing control example at accessing with preset periodic timing accompanying periodic output processing. The receiving device 3 monitors the passage of time after starting an examination and determines whether preset periodic transmission timing has come, as needed (step S11). Then, when the transmission timing comes (step S11, Yes), the transmission/output unit 31 generates packet data based on inside-subject image data picked up by the capsule endoscope 2 at the specific timing (step S12). Further, by obtaining address information, of the mobile phone 8 of a health care professional in charge set and registered in advance, from the storage unit 27 and accessing the mobile phone 8 corresponding to the address, the packet data is transmitted/output to the mobile phone 8 via the mobile phone interface circuit 13, antenna 14, and mobile phone communication network 7 (step S13). This makes it possible to display inside-subject images picked up by the capsule endoscope 2 on the display unit 9 of the mobile phone 8 of the health care professional in real time. Such processing is repeated periodically.

According to this example, a health care professional in charge can periodically perform real-time observation of inside-subject images under examination through the display unit 9 of the mobile phone 8 owned by the health care professional while remaining in a hospital, regardless of the location of the subject being examined by swallowing the capsule endoscope 2.

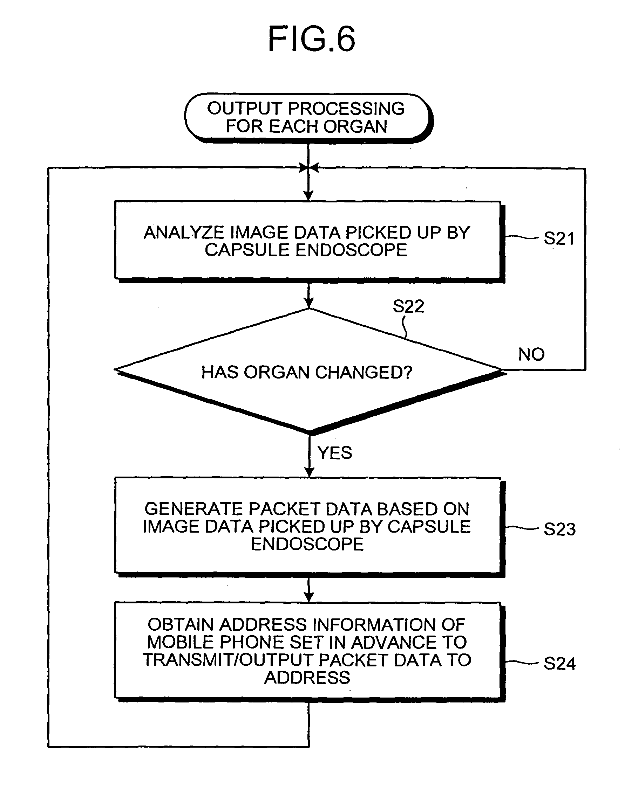

FIG. 6 is an outline flowchart showing a processing control example when accessing at timing when an imaged organ changes accompanying output processing for each organ. The receiving device 3 monitors any change after starting an examination through the organ determination unit 34 by analyzing image data picked up by the capsule endoscope 2 (step S21) to determine whether an imaged organ has changed, as needed (step 22). Then, if the imaged organ changes (step S22, Yes), the transmission/output unit 31 generates packet data based on inside-subject image data picked up by the capsule endoscope 2 at timing when the imaged organ changes (step S23). Further, by obtaining address information, of the mobile phone 8 of a health care professional in charge set and registered in advance, from the storage unit 27 and accessing the mobile phone 8 corresponding to the address, the packet data is transmitted/output to the mobile phone 8 via the mobile phone interface circuit 13, antenna 14, and mobile phone communication network 7 (step S24). This makes it possible to display inside-subject images picked up by the capsule endoscope 2 on the display unit 9 of the mobile phone 8 of the health care professional in real time. Such processing is repeated each time the imaged organ changes.

According to this example, a health care professional in charge can perform real-time observation of inside-subject images under examination through the display unit 9 of the mobile phone 8 owned by the health care professional while remaining in a hospital each time the organ changes, regardless of the location of the subject being examined by swallowing the capsule endoscope 2. Particularly, when arrival of the capsule endoscope 2 at the small intestine is confirmed while the subject is still in the hospital, the health care professional in charge can give permission to the subject to leave the hospital building or when arrival of the capsule endoscope 2 at the large intestine is confirmed while the subject is at some place outside the hospital building, considering that the examination is almost complete after passing through the small intestine, the health care professional in charge can instruct the subject to return to the hospital.

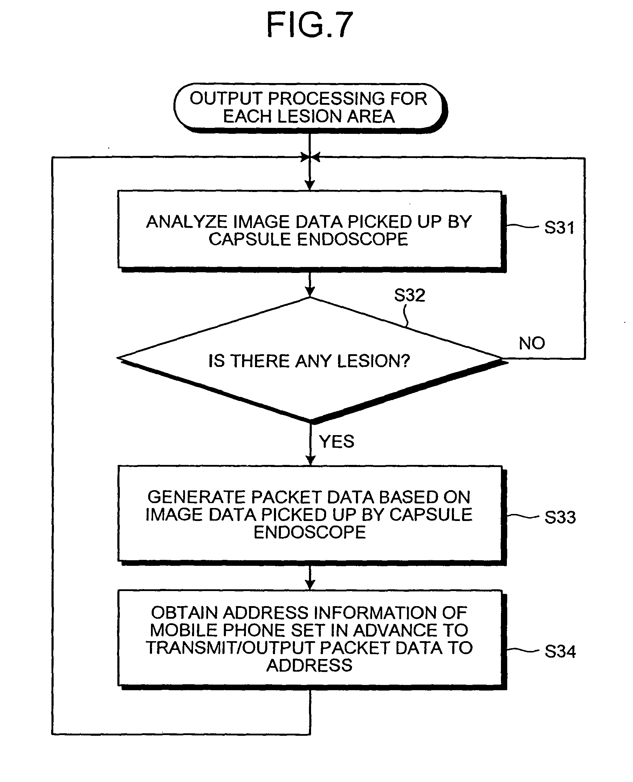

FIG. 7 is an outline flowchart showing a processing control example when accessing at timing when a lesion area is detected accompanying output processing for each lesion area. The receiving device 3 analyzes image data picked up by the capsule endoscope 2 (step S31) after starting an examination to determine whether there is any lesion area, as needed (step S32). Then, if a lesion area is detected (step S32, Yes), the transmission/output unit 31 generates packet data based on inside-subject image data picked up by the capsule endoscope 2 at timing when a lesion area is detected (step S33). Further, by obtaining address information, of the mobile phone 8 of a health care professional in charge set and registered in advance, from the storage unit 27 and accessing the mobile phone 8 corresponding to the address, the packet data is transmitted/output to the mobile phone 8 via the mobile phone interface circuit 13, antenna 14, and mobile phone communication network 7 (step S34). This makes it possible to display inside-subject images picked up by the capsule endoscope 2 on the display unit 9 of the mobile phone 8 of the health care professional in real time. Such processing is repeated each time a lesion area is detected.

According to this example, a health care professional in charge can perform real-time observation of inside-subject images under examination through the display unit 9 of the mobile phone 8 owned by the health care professional while remaining in a hospital each time a lesion area is detected, regardless of the location of the subject being examined by swallowing the capsule endoscope 2. Particularly, the health care professional in charge can determine severity of a lesion is and the like by observing image data of a lesion area and use the data as an aid to instruct the subject to return to the hospital or to continue the examination at rest as far as possible depending on the severity.

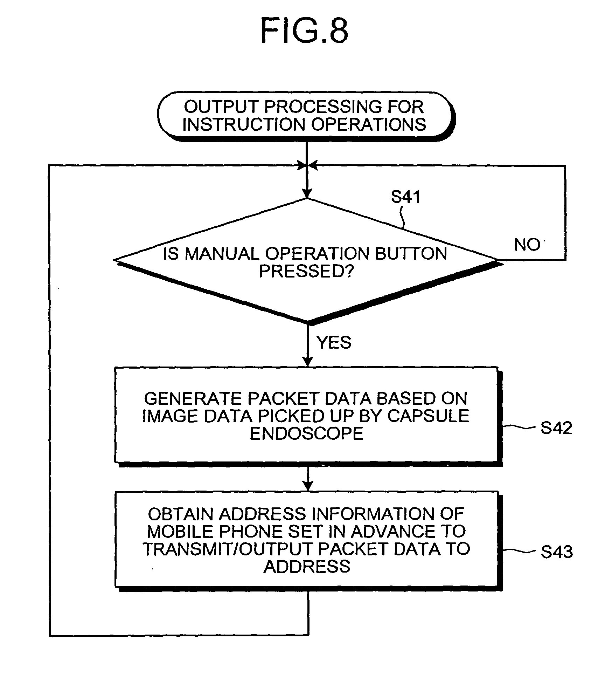

FIG. 8 is an outline flowchart showing a processing control example when accessing at timing when the manual operation button 16 of the receiving device 3 is pressed accompanying output processing for instruction operations. The receiving device 3 determines whether the manual operation button 16 is pressed after starting an examination, as needed (step S41). Then, if the manual operation button 16 is pressed (step S41, Yes), the transmission/output unit 31 generates packet data based on inside-subject image data picked up by the capsule endoscope 2 at timing when the button is pressed (step S42). Further, by obtaining address information, of the mobile phone 8 of the subject himself (herself) set and registered in advance, from the storage unit 27 and accessing the mobile phone 8 corresponding to the address, the packet data is transmitted/output to the mobile phone 8 via the mobile phone interface circuit 13, antenna 14, and mobile phone communication network 7 (step S43). This makes it possible to display inside-subject images picked up by the capsule endoscope 2 on the display unit 9 of the mobile phone 8 of the subject himself (herself) in real time. Such processing is repeated each time the manual operation button 16 is pressed.

According to this example, the subject himself (herself) can always perform real-time observation of inside-subject images under examination through the display unit 9 of the mobile phone 8 by accessing the mobile phone 8 owned by the subject from the receiving device 3 being carried by the subject, regardless of the location of the subject being examined by swallowing the capsule endoscope 2. Particularly, real-time observation can be performed by one-touch operation of pressing the manual operation button 16 of the receiving device 3 without accessing from the mobile phone 8. Nowadays, the mobile phone 8 has become an indispensable device for everyday life and, by simply carrying the mobile phone 8 and receiving device 3, real-time observation by the subject himself (herself) becomes possible.

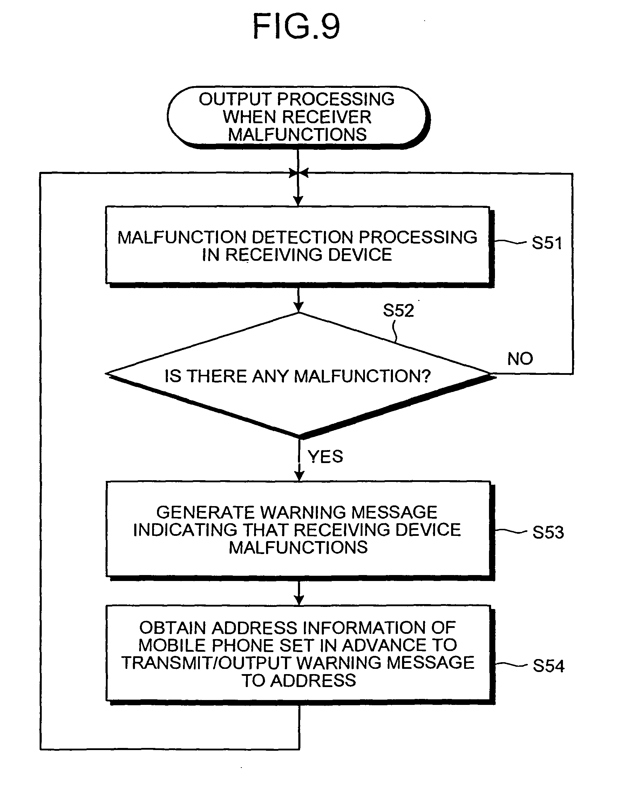

FIG. 9 is an outline flowchart showing a processing control example when accessing at timing when a malfunction is detected in the receiving device 3 accompanying output processing when a receiver malfunctions. The receiving device 3 performs malfunction detection processing inside the receiving device 3 after starting an examination through the receiver malfunction detection unit 36 as needed (step S51) to determine whether the receiving device 3 functions normally (step S52). Then, if a malfunction of the receiving device 3 is detected (step S52, Yes), the warning output unit 32 generates a warning message indicating that the receiving device 3 malfunctions at timing when a malfunction is detected (step S53). Further, by obtaining address information, of the mobile phone 8 of a health care professional in charge set and registered in advance, from the storage unit 27 and accessing the mobile phone 8 corresponding to the address, the warning message is transmitted/output to the mobile phone 8 via the mobile phone interface circuit 13, antenna 14, and mobile phone communication network 7 (step S54). This makes it possible to display a warning message indicating that the receiving device 3 malfunctions on the display unit 9 of the mobile phone 8 of the health care professional in real time. Such processing is repeated each time a malfunction inside the receiving device 3 is detected.

According to this example, a health care professional in charge can instantly know that a malfunction of the receiving device 3 occurred through the display unit 9 of the mobile phone 8 owned by the health care professional while remaining in a hospital, regardless of the location of the subject being examined by swallowing the capsule endoscope 2. Particularly, by knowing a malfunction of the receiving device 3, the health care professional can determine how severe the malfunction is and, depending on the severity, can instruct the subject to return to the hospital or stop the examination.

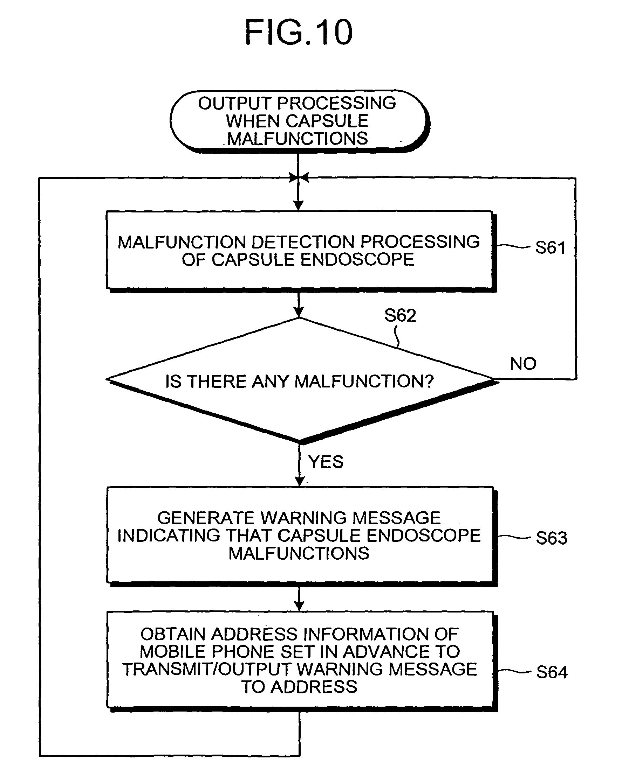

FIG. 10 is an outline flowchart showing a processing control example when accessing at timing when a malfunction is detected in the capsule endoscope 2 accompanying output processing when a capsule malfunctions. The receiving device 3 performs malfunction detection processing of the capsule endoscope 2 after starting an examination through the capsule malfunction detection unit 37 as needed (step S61) to determine whether the capsule endoscope 2 functions normally (step S62). Then, if a malfunction of the capsule endoscope 2 is detected (step S62, Yes), the warning output unit 32 generates a warning message indicating that the capsule endoscope 2 malfunctions at timing when a malfunction is detected (step S63). Further, by obtaining address information, of the mobile phone 8 of a health care professional in charge set and registered in advance, from the storage unit 27 and accessing the mobile phone 8 corresponding to the address, the warning message is transmitted/output to the mobile phone 8 via the mobile phone interface circuit 13, antenna 14, and mobile phone communication network 7 (step S64). This makes it possible to display a warning message indicating that the capsule endoscope 2 malfunctions on the display unit 9 of the mobile phone 8 of the health care professional in real time. Such processing is repeated each time a malfunction inside the capsule endoscope 2 is detected.

According to this example, a health care professional in charge can instantly know that a malfunction of the capsule endoscope 2 occurred through the display unit 9 of the mobile phone 8 owned by the health care professional while remaining in a hospital, regardless of the location of the subject being examined by swallowing the capsule endoscope 2. Particularly, by knowing a malfunction of the capsule endoscope 2, the health care professional can determine how severe the malfunction is and, depending on the severity, can instruct the subject to return to the hospital or stop the examination.

### (Modification 1)

In the above embodiment, radio communication between the receiving device 3 and mobile phone 8 is performed using the mobile phone interface circuit 13, antenna 14, and mobile phone communication network 7, but instead of the mobile phone 8, various communication terminal devices equipped with a display unit that can display images such as a notebook computer, workstation, or PDA (Personal Digital Assistance) that can use the mobile phone communication network 7 may be used.

### (Modification 2)

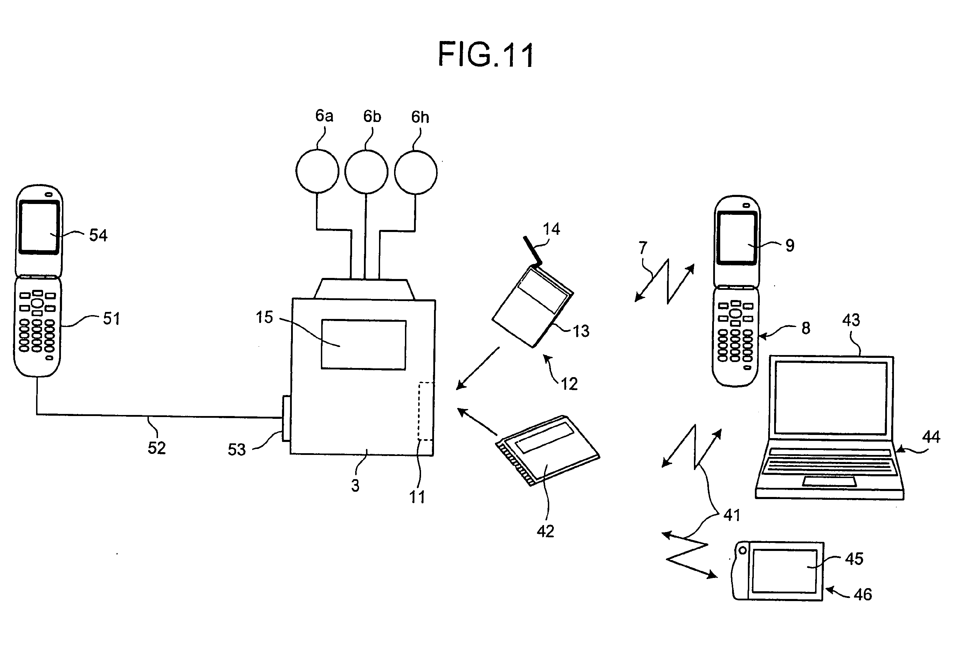

FIG. 11 is a schematic diagram showing the configuration of the receiving device 3, which can communicate by radio, of a Modification 2. The mobile phone communication network 7 is used in the above embodiment, but if the subject remains in the hospital until an examination is completed, a wireless LAN (Local Area Network) 41 constructed in the hospital may be used instead, and the receiving device 3 may be adapted to communicate by radio with a communication terminal device such as a notebook computer 44 having a display unit 43 that can display images and a PDA 46 having a display unit 45 by equipping the receiving device 3 with a wireless LAN interface 42 that is wireless LAN compliant and to which a specific address is specified. The communication terminal device in this case may be a workstation or the like. Also, by making the wireless interface card 12 and the wireless LAN interface 42 freely mountable to the slot 11 by selecting, for example, a CF card compliant wireless LAN interface as the wireless LAN interface 42, a suitable interface may be used depending on the time when the mobile phone communication network 7 is used or when the wireless LAN 41 is used.

According to this modification, a health care professional in charge can perform real-time observation of inside-subject images under examination through the display unit 43 or 45 by accessing the receiving device 3 being carried by the subject at a desired time from the notebook computer 44 or the PDA 46, regardless of the location in the hospital of the subject being examined by swallowing the capsule endoscope 2. Also, the health care professional in charge can perform real-time observation of inside-subject images under examination through the display unit 43 or 45 of the notebook computer 44 or PDA 46 via access from the receiving device 3, regardless of the location in the hospital of the subject being examined by swallowing the capsule endoscope 2.

The receiving device 3 may be equipped with a USB (Universal Serial Bus) port 53 as a connection port for connecting a mobile phone 51 of the subject himself (herself) via a USB cable 52 and inside-subject images picked up by the capsule endoscope 2 may be made to transmit/output to the mobile phone 51 via the USB port 53 and USB cable 52 to be displayed on the display unit 54 of the mobile phone 51 when the mobile phone 51 is connected to the USB port 53.

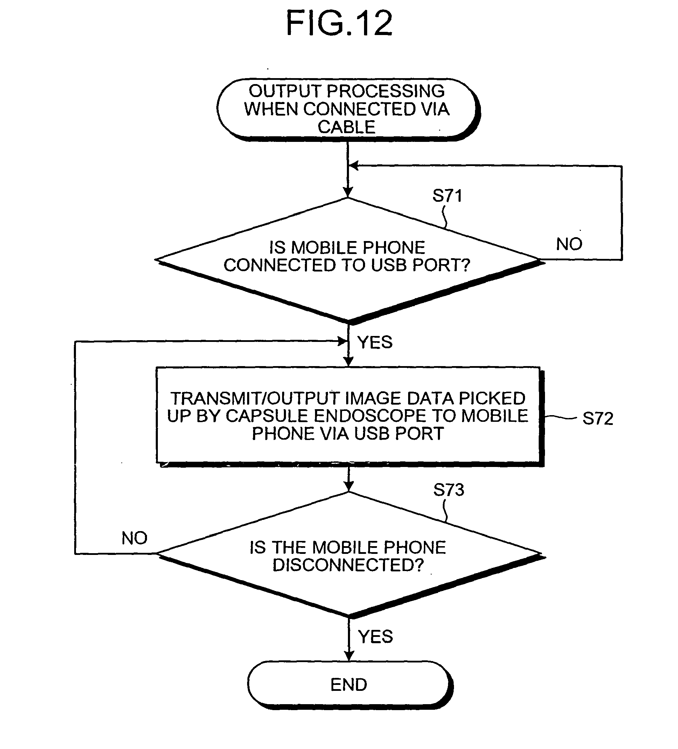

FIG. 12 is an outline flowchart showing an output processing example when connected via a cable. The control unit 28 determines whether the mobile phone 51 is connected to the USB port 53 as needed (step S71) and if the mobile phone 51 is connected (step S71, Yes), an output unit transmits/outputs inside-subject image data picked up by the capsule endoscope 2 to the mobile phone 51 via the USB port 53 (step S72). This processing is repeated until the mobile phone 51 is disconnected (step S73, Yes).

According to this modification, by using the mobile phone 51 owned by the subject, the subject can perform communication between the receiving device 3 being carried by the subject and the mobile phone 51 of the subject regardless of the location of the subject being examined by swallowing the capsule endoscope 2 and thus, can perform real-time observation of inside-subject images under examination through the display unit 54 of the mobile phone 51 owned by the subject regardless of where the subject is located. Particularly, the mobile phone 51 has nowadays become an indispensable device for everyday life and, by simply carrying the mobile phone 51 and receiving device 3, real-time observation by the subject himself (herself) becomes possible, eliminating inconvenience of carrying a dedicated simplified image display device.

### INDUSTRIAL APPLICABILITY

As has been described, a receiving device according to the present invention is useful for receiving a radio signal transmitted from a body-insertable device moving inside the subject such as a capsule endoscope and is appropriate particularly when performing real-time observation of inside-subject images being examined by taking a capsule endoscope or monitoring for malfunctions of the receiving device itself and the body-insertable device regardless of where the subject is located.

## Claims

1. A receiving device capable of receiving a radio signal transmitted from a body-insertable device moving inside a subject, allowing the subject to carry, the receiving device comprising:
a radio communication interface that enables radio communication via a radio communication network with a communication terminal device having a display unit capable of displaying an image.

2. The receiving device according to claim 1, wherein the radio communication interface is a mobile phone interface.

3. The receiving device according to claim 1, wherein the radio communication interface is a wireless LAN interface.

4. The receiving device according to any one of claims 1 to 3, comprising a mounting unit to which the radio communication interface adapted to a type of target communication terminal device can freely be mounted.

5. The receiving device according to any one of claims 1 to 4, comprising a transmission/output unit that transmits/outputs inside-subject image data picked up by the body-insertable device, to the communication terminal device via the radio communication interface with an access from the communication terminal device.

6. The receiving device according to any one of claims 1 to 5, comprising:
an access unit that accesses the communication terminal device of a preset specific address; and
a transmission/output unit that transmits/outputs inside-subject image data picked up by the body-insertable device to the communication terminal device via the radio communication interface during an access by the access unit.

7. The receiving device according to claim 6, wherein the access unit accesses the communication terminal device of the specific address at specific timing.

8. The receiving device according to claim 7, wherein the specific timing is periodic timing.

9. The receiving device according to claim 7, comprising an organ determination unit that determines any change of an imaged organ by performing image processing of inside-subject image data picked up by the body-insertable device, wherein
the specific timing is a timing when the organ imaged by the body-insertable device changes according to a determination of the organ determination unit.

10. The receiving device according to claim 7, comprising a lesion area determination unit that determines whether there is any lesion area by performing image processing of inside-subject image data picked up by the body-insertable device, wherein
the specific timing is a timing when a lesion area is detected according to a determination of the lesion area determination unit.

11. The receiving device according to claim 7, comprising a manual operation button for instructing transmission/output of inside-subject image data, wherein
the specific timing is a timing when the manual operation button is operated.

12. The receiving device according to any one of claims 1 to 11, comprising:
an access unit that accesses the communication terminal device of a preset specific address; and
a warning output unit that transmits/outputs warning information to the communication terminal device via the radio communication interface during access by the access unit.

13. The receiving device according to claim 12, comprising a malfunction detection unit that detects whether there is any malfunction of the receiving device, wherein
the access unit accesses the communication terminal device of the specific address when a malfunction is detected by the malfunction detection unit, and
the warning output unit transmits/outputs warning information, indicating that the receiving device malfunctions, to the communication terminal device.

14. The receiving device according to claim 12, comprising a malfunction detection unit that detects whether there is any malfunction of the body-insertable device, wherein
the access unit accesses the communication terminal device of the specific address when a malfunction is detected by the malfunction detection unit, and
the warning output unit transmits/outputs warning information, indicating that the body-insertable device malfunctions, to the communication terminal device.

15. A receiving device that can receive a radio signal transmitted from a body-insertable device moving inside a subject, allowing the subject to carry, the receiving device comprising:
a connection port for connecting a mobile phone via a cable; and
an output unit that transmits/outputs inside-subject image data picked up by the body-insertable device to the mobile phone via the connection port when the mobile phone is connected to the connection port.
